# EUROPEAN PATENT APPLICATION

(11) **EP 2 471 461 A1**
(43) Date of publication of application: **04.07.2012**
(21) Application number: 11195721.3
(22) Date of filing: 23.12.2011
(51) Int. Cl.: A61B 7/00, A61B 7/02, A61B 7/04

(54) **An integrated monitoring device arranged for recording and processing body sounds from multiple sensors**

(30) Priority: 29.12.2010 US 428188 P
(71) Applicant: Stichting IMEC Nederland, 5656 AE Eindhoven (NL)
(72) Inventor: Penders, Julien, 4020 Liège (BE); Grundlehner, Bernard, 5616 NZ Eindhoven (NL)
(74) Representative: Sarlet, Steven Renaat Irène

(57) **Abstract**

Integrated body sound monitoring system arranged for recording and processing body sounds such as respiratory sounds. The system comprises a number of microphones arranged for recording a body sound signal that represents a mixture of different body sounds; a processing block that is arranged for processing the body sound signal and comprises electronic components for locally storing, processing and analyzing the body sound signal prior to transmittal to an external device; and a radio that is arranged for transmitting data related to the body sound signal to the external device.

## Description

### FIELD

The invention relates generally to monitoring systems. More in particular, it relates to an integrated monitoring device arranged for recording and processing body sounds. The invention also relates to an integrated body sound monitoring system arranged for recording and processing body sound signals. The invention further relates to a method for monitoring body sounds. The method further relates to a method of placement of a plurality of integrated body sound monitoring systems.

### BACKGROUND

Monitoring of a body sound has since long been recognised as having significance for assessing health of an individual. Medical practitioners have for example used a stethoscope for listening to heart sounds. By experience and by training, medical practitioners have been able to use the monitored body sound for health assessment.

Recently, more elaborate devices were developed for monitoring a body sound. For example, in WO2008/018069 a microphone matrix is presented for recording a body sound. Additionally, in US 6394967 a system is presented arranged for displaying lung sounds and performing diagnosis based on lung signals.

Typically, these recent known solutions are bulky systems and designed for measuring a particular body sound. Furthermore, the recorded sounds are transmitted via conductors or wires to be processed and analyzed. Hence, these known systems are typically used in a hospital or on another location dedicated to said monitoring. Thus, these known systems usually require the presence of the individual on a dedicated monitoring location.

### SUMMARY

There is needed a simple integrated autonomous system which can provide the user with physiological and psychological health information extracted from a multiple of recorded body sounds.

The invention presents an integrated body sound monitoring system arranged for recording and processing body sounds such as respiratory sounds. The system comprises a number of microphones arranged for recording a body sound signal, whereby the body sound signal (simply body sound) is a mixture of different sounds. The system further comprises a processing block and a radio. The processing block is arranged for (pre-)processing the body sound. The radio is arranged for transmitting data related to the body sound to an external device. The processing block further comprises electronic components for locally storing, processing and analyzing (by means of an analyzing block) the body sound prior to transmittal to said external device.

A simple and integrated system is presented to provide the user with physiological and psychological health information extracted from a multiple of recorded body sounds.

Accordingly, a method is presented for monitoring a body sound, by means of an integrated body sound monitoring system.

It may thus be clear that, according to an aspect of the invention, there is provided an integrated body sound monitoring system arranged for recording and processing body sound signals, wherein the system comprises:
- a number of microphones, e.g. at least one microphone or a plurality of microphones, arranged for recording a body sound signal that represents a mixture of different body sounds; - a processing block that is arranged for processing the body sound signal and comprises electronic components for locally storing, processing and/or analyzing the body sound signal, preferably prior to transmittal to an external device; and, preferably, - a radio that is arranged for transmitting data related to the body sound signal to the external device. Thus, a relatively simple and integrated system may be presented to provide the user with physiological and psychological health information extracted from a multiple of recorded body sound signals. The radio may enable transmitting said data from a variety of locations. Hence, the system can be used on a variety of locations. As a result, the system may be used continuously, e.g. during a number of days, weeks, or months, without requiring presence of the user at a dedicated monitoring locating such as a hospital. Additionally, the radio may enable placement of the system on various positions in a close vicinity of, or at, the human body.

The radio may be arranged for providing a wireless connection between the system and the external device, and/or to further systems. Said radio may, in an embodiment, enable a combination of a plurality of systems, without the need for complicated wiring between the systems. The systems may be distributed over the human body. Hence, the systems may be placed in a close vicinity of, or at, mutually different positions on a human body. Said positions may be relatively far away from each other, e.g. more than 0.3 meter, more than 0.6 meter, or more than 0.9 meter. Preferably, distances are measured along a skin of the human body. By means of said systems, body sound signals originating from different parts of the human body may be recorded. In an embodiment, a single system may be used for recording and processing body sound signals.

A system, in particular a microphone of the system, may be placed so that it is dedicated to record a body sound signal that substantially, e.g. predominantly, represents a specific type of body sound, e.g. a speech sound, a lung sound, a heart sound, or a gastrointestical tract sound. Preferably, in use, a system, in particular a microphone of the system, may be placed so that it is dedicated to record a body sound signal that substantially, e.g. predominantly, represents a single type of body sound. Hence, one type of body sound may, preferably, dominate the mix of body sounds as a result of the placement. Alternatively, a system is arbitrarily placed. The number of microphones may optionally be used to capture, i.e. to record, body sound signals from different body sound sources. Analyzing the body sound signal, or data related thereto, may optionally enable a quick determination of physiological and/or psychological health information. Alternatively or additionally, analyzing the body sound signal, or data related thereto, may optionally enable physical and psychological health monitoring on long term.

In an embodiment, the mixture of body sounds comprises at least one, at least two, or at least three sounds of the group comprising: a heart sound, a lung sound, a speech sound and a gastrointestical tract sound. Preferably, the mixture of body sounds comprises at least the speech sound. Preferably, the mixture of body sounds comprises at least the heart sound, the lung sound, and the speech sound.

In an embodiment, the processing block comprises an analyzing block for analyzing the body sound signal, preferably prior to transmittal to the external device. Preferably, the analyzing block comprises means for data fusion arranged for combining a plurality of recorded body sound signals from the microphones. Said data fusion preferably comprises combining body sound signals obtained from at least two microphones, or data related to the body sound signals obtained from the at least two microphones. Data fusion may be carried out before or after processing, or may e.g. be part of the processing. Said at least two microphones may be comprised by at least two systems or by a single system. Data fusion may comprise e.g. adaptive filtering with a reference input, pattern recognition, heart sound analysis in conjunction with breathing cycle state (for example for heart rate variability analysis), and/or combined speech and heart rate variability analysis for emotion monitoring. Preferably, the data fusion is used for reducing the impact of noise. Preferably, the data fusion is used for analyzing health conditions, by analyzing different recorded body sound signals, e.g.: heart sounds, lung sounds, speech sounds, and/or gastrointestical tract sounds. A result of the health analysis may, preferably, be used to provide feedback on a health status of an individual that may, in use of the system, be monitored.

Preferably, the analyzing block comprises means for analyzing health conditions arranged for analyzing the recorded body sound signals. The processing block, in particular the analyzing block, may for example be arranged for filtering or transforming the recorded body sounds. The processing block, in particular the analyzing block, may be arranged for performing filtering, wavelet transformation, beamforming, and/or acoustic holography, for analyzing the recorded body sound signals. Thus, preferably, the processing block is arranged for receiving the body sound signal that represents the mix of body sounds and for processing, in particular for preprocessing, the body sound signal. The processing block, in particular the analyzing block, preferably is provided with software components for carrying out such functions. Alternatively or additionally, the system may comprise a means for storing the body sounds. Hence, alternatively or additionally, the data may be transferred from said storing means to the external device.

In an embodiment, the external device is formed by a processor for further processing and/or analyzing the data related to the body sound signal. For example, in use, the data is transmitted to the processor for further processing and analyzing the body sound signal. The processor may for example be arranged for filtering or transforming the recorded body sounds. In particular, the processor may be arranged for performing any of the following actions: filtering, wavelet transformation, beamforming, acoustic holography, and/or another processing activity. The processor preferably is provided with software components for carrying out such functions.

Preferably, the processing and/or analyzing of the body sound signal, by means of the processing block, the analyzing block, the electronic components, and/or by means of the external device, e.g. the processor, comprises determining a wavelet transform and/or a fourier transform of the body sound signal or data related thereto. In particular, the processing and/or analyzing of the body sound signal may comprise determining a continuous wavelet transform and/or a short-time fourier transform of the body sound signal or data related thereto. The wavelet may e.g. be a Morlet wavelet or a Gaussian wavelet. Preferably, the processing and/or analyzing may comprise filtering the body sound signal or data related thereto. Experiments carried out by the inventors showed that such ways of processing and/or analyzing may yield useful results. In an embodiment, the system is arranged for providing the user with physiological and psychological health information extracted from a multiple of recorded body sounds signals or data related thereto.

Thereto the processing block and/or the external device may be arranged for determining physiological and/or psychological health information extracted from a multiple of recorded body sounds signals or data related thereto. Such extracting may comprise said processing and/or analyzing. Preferably, the processing block, the analyzing block, the electronic components, and/or the external device, e.g. the processor, are provided with software components for carrying out such functions.

It may be clear that, in an embodiment, part, or all, of the processing, analyzing, and/or storing may be carried out by means of the system. Optionally, part, or all, of the processing, analyzing, and/or storing may be carried out by means of the external device.

In an embodiment, at least one of the number of microphones comprises a piezoelectric element that comprises polyvinylidene fluoride, and preferably is substantially made of polyvinylidene fluoride. Preferably, the piezoelectric element is formed as a sheet. Preferably, the element is flexible. Such flexibility may enable placement of the microphone in a close vicinity of, or at, a positions on a human body. Such flexibility may in particular be appreciated when placing a microphone in a vicinity of a throat of the human body, as a curvature of the human body may be large near said throat. Hence, the piezoelectric element that comprises polyvinylidene fluoride may be especially appreciated as part of a contact microphone.

In an embodiment, the system comprises a number of acoustic microphones. With the term 'acoustic microphone' a microphone may be meant that is arranged to pick up sounds from the air. Hence, an acoustic microphone may be different from a contact microphone or a surface microphone. Alternatively or additionally, the number of microphones comprises at least one acoustic microphone and at least one contact microphone. Preferably, the system comprises sensing means arranged for sensing at least one physiological signal such as a biopotential, a tissue impedance, and/or a temperature. As a result, the system may be arranged, by means of the physiological signals, to estimate or predict the health condition of a user and/or to help to improve a quality of the body sound signal.

The external device can e.g. be a processor, a mobile terminal, or a display unit.

In an embodiment, the system is arranged for providing the user with physiological and psychological health information extracted from a multiple of recorded body sounds signals.

In an embodiment, the system is arranged as a patch arranged for placement in a close vicinity of a person.

According to an aspect, the system is a distributed system. The distributed system may comprise a plurality of integrated body sound monitoring units according to the invention. A unit according to the invention may be formed by a system, preferably not being a distributed system, according to the invention. The unit may e.g. be formed as the patch. Said units may be arranged for placement in a close vicinity of a person, preferably for placement at several positions of a human body, and for forming a network, wherein each system, in use, records at least one body sound signal that represents a mixture of body sounds and processes, e.g. digitizes, the at least one body sound signal, wherein, in use, the recorded and processed body sound signals of each body sound system are transmitted to the external device.

According to an aspect, there is provided a plurality of integrated body sound monitoring systems, in particular a plurality of integrated body sound monitoring units, according to the invention, being arranged for placement in a close vicinity of a person, preferably for placement at several positions of a human body, and for forming a network, wherein each system, in use, records at least one body sound signal that represents a mixture of body sounds and processes, e.g. digitizes, the at least one body sound signal, wherein, in use, the recorded and processed body sound signals of each body sound system are transmitted to the external device. A part or all of the plurality of systems may be arranged as a patch.

In an embodiment, at least one of the systems, in particular at least one of the units, is arranged to monitor environment-, context- or motion-related sounds, by having, in use, a microphone placed on such a location that a speech sound, a lung sound, and a cardio-vascular sound are not recorded or at least not recorded with a high attenuation. Thus, in an embodiment, at least one of the systems may be dedicated to recording context- or motion-related sounds. In an embodiment, at least one of the systems is placed for substantially receiving context- or motion-related sounds.

In an embodiment, a system, in particular a unit, of the plurality of systems, in particular of the plurality of units, is arranged for receiving data coming from other systems of the plurality of systems. Preferably, this system comprises an analyzing block that is comprised by the processing block. Preferably, the analyzing block is arranged for combining the recorded and processed body sound signals by said system of the plurality of systems with the received signals from other systems of the plurality of systems.

It may further be clear that, according to an aspect of the invention, a method is provided for monitoring body sounds, by means of an integrated body sound monitoring system according to the invention or a plurality of integrated body sound monitoring systems according to the invention. The method preferably comprises providing an integrated body sound monitoring system arranged for recording and processing body sound signals. The method preferably comprises recording a body sound signal that represents a mixture of different body sounds, by means of a number of microphones, e.g. at least one microphone or a plurality of microphones, comprised by the system. The method preferably comprises, by means of a processing block comprised by the system, processing the body sound signal. The method preferably comprises locally storing, processing and/or analyzing the body sound signal by means of electronic components of the processing block, preferably prior to transmittal to an external device. The method further preferably comprise transmitting, by means of a radio comprised by the system, data related to the body sound signal to the external device.

According to an aspect of the invention, there is provided a method of placement of a plurality of integrated body sound monitoring systems in a close vicinity of, or at, mutually different positions of a human body, wherein each of the systems comprises at least one microphone arranged for recording a body sound signal that represents a mixture of different body sounds, and further comprises a processing block that is arranged for processing the body sound signal and comprises electronic components that are arranged for locally storing, processing and/or analyzing the body sound signal, preferably prior to transmittal to an external device, and further comprises a radio that is arranged for transmitting data related to the body sound signal to the external device, wherein the method comprises at least one, at least two, or at least three, preferably four, steps of the following group of four steps: - placing the at least one microphone of at least one of the systems at a position, preferably in a vicinity of a heart of the human body, that is suitable for substantially recording a heart sound; - placing the at least one microphone of at least one of the systems at a position, preferably in a vicinity of a lung of the human body, that is suitable for substantially recording a lung sound; - placing the at least microphone of at least one of the systems at a position, preferably in a vicinity of a throat of the human body, that is suitable for substantially recording a speech sound; and - placing the at least one microphone of at least one of the systems at a position, preferably in a vicinity of an abdomen of the human body, that is suitable for substantially recording a gastrointestical tract sound. Preferably, the steps are carried out by mutually separate systems. Hence, a system used in one of the steps is preferably not used in another one of the steps. Optionally, said term "close vicinity" may e.g. be interpreted as within a distance smaller than 0.2, 0.1, or 0.05 meter. Optionally, said term "in a vicinity of" may e.g. be interpreted as within a distance smaller than 0.3, 0.2, or 0.1 meter. Optionally, the term "locally storing" may optionally be interpreted as storing in the system itself, e.g. away from the external device.

Such placement may enable recording at least one, at least two, or at least three body sound signals that each represent a mixture of body sounds. As a result of the placement, each mixture may be dedicated to a specific body sound, i.e. the heart sound, the lung sound, the speech sound, the gastrointestical sound, or another body sound. Hence, the placement enables a combination of recording of different body sounds. Experiments carried out by the inventors showed that a quality of recorded sound may be enhanced by placement according to said method. By means of the radio of the systems, such placement may be accomplished without the need for complicated wiring between the systems.

In an embodiment, the method comprises: - placing the at least one microphone of at least one of the systems at a position, preferably in a vicinity of a throat of the human body, that is suitable for substantially recording a speech sound. Preferably, the method further comprises placing the at least one of the microphones of at least one the systems at a position, preferably in a vicinity of a heart of the human body, that is suitable for substantially recording a heart sound; and - placing the at least one of the microphones of least one of the systems at a position, preferably in a vicinity of a lung of the human body, that is suitable for substantially recording a lung sound. Thus, in this embodiment, the recording of speech sound may be enabled. It was recognized by the inventors that recording of speech sound may improve and/or enable an analysis of an emotion of a person. In particular, the combination of the speech sound with the recording of the heart sound and the lung sound may enable an analysis of said person with a relatively high quality.

The method may further comprise recording the heart sound by means of the at least one microphone of the at least one the systems placed at the position that is suitable for substantially recording the heart sound, may comprise recording the lung sound by means of the at least one microphone of the at least one the systems placed at the position that is suitable for substantially recording the lung sound, may comprise recording the speech sound by means of the at least one of the microphones of the at least one the systems placed at the position that is suitable for substantially recording the speech sound, and/or may comprise recording the gastrointectical sound by means of the at least one of the microphones of the at least one of the systems placed at the position that is suitable for substantially recording the gastrointestical tract sound.

Thus, there may be provided a method of placement of a plurality of integrated body sound monitoring systems in a close vicinity of, or at, mutually different positions of a human body, wherein each of the systems comprises at least one microphone arranged for recording a body sound signal that represents a mixture of different body sounds, and further comprise a processing block that is arranged for processing the body sound signal and comprises electronic components that are arranged for locally storing, processing and/or analyzing the body sound signal, preferably prior to transmittal to an external device, and further comprises a radio that is arranged for transmitting data related to the body sound signal to the external device, wherein the method comprises at least one, at least two, or at least three, preferably four, steps of the following group of four steps: - placing the at least one microphone of at least one of the systems at a position for substantially recording a heart sound, preferably at a position in a vicinity of a heart of the human body; - placing the at least one microphone of at least one of the systems at a position for substantially recording a lung sound, preferably at a position in a vicinity of a lung of the human body; -placing at least one of the microphones of at least one of the systems at a position for substantially recording a speech sound, preferably at a position in a vicinity of a throat of the human body; and - placing the at least one of the microphones of at least one of the systems at a position for substantially recording a gastrointestical tract sound, preferably at a position in a vicinity of an abdomen of the human body.

Preferably, the method further comprises, by means of the processing block, the analyzing block, the electronic components and/or by means of the external device, storing, analyzing, and/or processing the recorded body sound signals.

The processing may, in an embodiment, comprise combining at least two, preferably at least three, recorded body sound signals for reducing noise and/or enhancing quality of the body sound signals. In an embodiment wherein a system comprises a plurality of microphones, said processing may e.g. comprise combining the body sound signals recorded by means of different microphones, preferably for reducing noise and/or enhancing quality of the body sound signals. Preferably, the method further comprising transmitting data related to the recorded body sound signals to the external device. In an embodiment, the external device is formed by a processor for further processing and/or analyzing the data related to the body sound signal.

The method may comprise providing the user with physiological and psychological health information extracted from a multiple of recorded body sounds signals or data related thereto. Thereto the method may comprise, by means of the processing block and/or the external device, determining physiological and/or psychological health information extracted from a multiple of recorded body sounds signals or data related thereto. Determining health information may comprise comparing body sound signals or data related to body sound signals with predetermined comparison data.

Any described aspect may optionally be combined with a described embodiment, example, variation, a preferred or optional feature, or another feature.

### BRIEF DESCRIPTION OF THE DRAWINGS

Presently preferred embodiments are described below in conjunction with the appended drawing figures, wherein like reference numerals refer to like elements in the various figures, and wherein:
Figure 1 illustrates a first embodiment of an integrated system described in the present disclosure.
Figure 2 illustrates a second embodiment of an integrated system described in the present disclosure.
Figure 3 illustrates a network of integrated systems according to an embodiment.
Figure 4 illustrates a network of integrated systems according to another embodiment.
Figure 5 shows a block diagram illustrating the present system and algorithms.
Figure 6 schematically shows a plurality of systems placed on a human body.

### DESCRIPTION OF THE INVENTION

The present invention will be described with respect to particular embodiments and with reference to certain drawings but the invention is not limited thereto. The drawings described are only schematic and are nonlimiting. In the drawings, the size of some of the elements may be exaggerated and not drawn on scale for illustrative purposes.

Furthermore, the terms first, second, third and the like in the description, are used for distinguishing between similar elements and not necessarily for describing a sequential or chronological order. The terms are interchangeable under appropriate circumstances and the embodiments of the invention can operate in other sequences than described or illustrated herein.

Moreover, the terms top, bottom, over, under and the like in the description and the claims are used for descriptive purposes and not necessarily for describing relative positions. The terms so used are interchangeable under appropriate circumstances and the embodiments of the invention described herein can operate in other orientations than described or illustrated herein.

The term "comprising", used in the claims, should not be interpreted as being restricted to the means listed thereafter; it does not exclude other elements or steps. It needs to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting of only components A and B. It means that with respect to the present invention, the only relevant components of the device are A and B.

The body emits sounds from many different sources which can be used for monitoring of body functions and assessment of health - including physiological and psychological health - on a continuous basis. The body sounds can typically be classified in four or five categories:
- Voice and speech
- Heart and vascular sounds, cardiovascular sounds
- Respiratory sounds
- Gastrointestical sounds
- Motion-related sounds

It was recognized that heart sounds may result from the interplay of the dynamic events associated with the contraction and relaxation of the atria and ventricles, valve movements, and blood flow. During the systolic and the diastolic phase of the cardiac cycle, audible sounds are produced from opening and closing of the heart valves, the flow of blood in the heart, and the vibration of heart muscles. Usually, four heart sounds are generated in a cardiac cycle. The first and the second heart sound can be easily heard in a normal heart through a stethoscope placed on the chest. The normal third heart sound is audible in children and adolescents but not in most adults. The fourth heart sound can normally be detected only with high-sensitivity sensors. All other sounds are abnormal and called murmurs, in many cases indicating valve malfunctioning.

A first type of heart sound (S1) may occur at the onset of ventricular systole. It may be most clearly heard at an apex of the heart, or near a fourth intercostal space along the left sternal border of the heart. It may lasts for an average period of 100 - 200 ms. It may have frequency components between 10 and 200 Hz.

A second type of heart sound (S2) may occur within a relatively short period once the ventricular diastole starts. The second type of heart sound may coincide with completion of a T-wave of an ECG. The second type of heart sound may be most clearly heard at the second or third intercostal space along the left sternal border. The second type of heart sound may comprise two components, related to respectively closure of the aortic and the pulmonary valves. Time between the components may vary with respiratory variations (e.g. larger during inspiration). S2 sounds may have higher frequencies than S1 sounds.

Lung sounds may be divided into Tracheal lung sounds, Vesicular lung sounds, Bronchial lung sounds, Bronchovesicular lung sounds and normal crackles.

Tracheal lung sounds may be heard over the trachea. They may be relatively loud and relatively high-pitched. The inspiratory and expiratory sounds may be more or less equal in length. Vesicular lung sounds may form the major normal breath sound and may be heard over most of the lungs.

They sound relatively soft and low-pitched. The inspiratory sounds may be longer in duration than the expiratory sounds.

Vesicular lung sounds may be harsher and slightly longer if there is rapid deep ventilation (for example after exercise) or in children who have thinner chest walls. Vesicular breath sounds may also be softer if the patient is frail, elderly, obese, or very muscular.

Bronchial lung sounds may be relatively loud and high-pitched. There may be a gap between the inspiratory and expiratory phases of respiration. The expiratory sounds may be longer than the inspiratory sounds. These sounds are typically heard over the trachea and the larynx (for example on the Manubrium).

Bronchovesicular sounds may be of intermediate intensity and pitch (in between vesicular and bronchial breath sounds). The inspiratory and expiratory sounds may be equal in length. They may be best heard in the 1st and 2nd intercostal space (anterior chest) and between the shoulder blades (scapulae, posterior chest).

Typical frequency ranges for lung sounds is from 60 Hz up to 2 kHz when recorded on the chest and up to 4 kHz when recorded over the trachea.

When the vocal cords are placed under tension by the surrounding musculature, air pressure from the lungs causes the vocal cords to vibrate. Vocal cord tension may be governed by a control input to the musculature. The vocal cords' periodic output can be described by a periodic pulse train, yielding a spectrum containing many harmonics, with a fundamental frequency called 'pitch'. Before puberty, pitch frequency for normal speech ranges between 150-400 Hz for both males and females. After puberty, the vocal cords of males usually undergo a physical change, which has the effect of lowering their pitch frequency to the range 80-160 Hz. The sound pressure signal thus produced enters the vocal tract, consisting of the mouth, tongue, teeth, lips and nasal cavity. The vocal tract serves to shape the spectrum of the vocal cords. Spreading the lips, bringing the teeth together, and bringing the tongue toward the front portion of the roof of the mouth produces the sound "ee." Rounding the lips, spreading the teeth, and positioning the tongue toward the back of the oral cavity produces the sound "oh." These variations typically result in a, by approximation, linear, time-invariant system that has a frequency response typified by several peaks, called formants. In short, the peaky spectrum that is created by the vocal cords is shaped by the vocal tract which will result in amplification of some frequencies and attenuation of the others.

When recording body sounds with a contact microphone of any kind, recorded speech sound characteristics may vary according to the location where the microphone is placed. An uncoloured spectrum with pitch and its harmonics will be apparent close to the vocal cords, whereas formants can be captured near the vocal tract (closer to the mouth).

The inventors recognized that microphone design and placement location may be chosen according to the measurement purpose, e.g. a body sound of interest, or application.

Generally, body sound may be captured in either one of the following manners:
1. By capturing air-borne sound;
2. By capturing sounds at hard structures, such as bones, or at highly vibrating soft structures of the body, such as the throat. This will be called 'body-borne' sound.

Air-borne sounds may typically comprise speech and environment sounds, such as other people talking, music, ambient noise, etc. Environmental sounds may be considered as noise if one is interested in the speech signal or speech sound of the user. The inverse may be true when studying environmental noise. Body-borne sounds may typically comprise heart sounds, vascular sounds and respiratory sounds. Non-desired sounds, for example related to motion can also be propagated as body-borne sounds. The cardiovascular scientist may treat respiratory and motion-related sounds as noise, whereas the respiratory specialist may tend to filter out the respiratory components from all the other sounds, which he or she may consider as undesired noise.

Up to now, problems related to noise have been solved in the following ways:
1. For air-borne sound, directional microphones have been used, in order to reduce as much as possible the sounds coming from other directions than the desired one. Such directionality can be realized by means of mechanical modification of the microphone housing, or by applying array technologies, where several sensors of the same type are lined up and recombined (often by means of digital signal processing techniques) to form a pattern with high sensitivity in the desired direction, lower sensitivity in other directions and very low sensitivity in a limited number of directions where interfering noises are located.
2. For body-borne sounds, sensors have been positioned on a spot where the sound of interest is most obviously detected.
3. Signal processing techniques have been applied to perform 'post-processing' on the captured signal (either air-borne or body-borne), which should remove as much as possible from the background noise while keeping the wanted signal unchanged. It is clear that these post-processing techniques might lead to loss of information, if the last condition is not fulfilled.

It is an aim of the invention to provide an integrated system solving following problems:
1. Providing a simple, integrated body sound monitoring system for providing the user with physiological and psychological health information extracted from a multiple of recorded body sounds.
2. The system can be used under ambulatory conditions, as a on-the-go device, capable of monitoring (recording and processing) noisy signals, comprising typically motion artifacts. Furthermore, the system can be used for longitudinal or long-term monitoring (involving the repeated observation or examination of a set of subjects over time with respect to one or more study variables). Health conditions can be monitored over time by looking at trends. Recorded body sound signals, or data related thereto, e.g. derive therefrom, may be compared with comparison data. Such comparison data may e.g. represent a healthy individual, a sick individual, or a an individual that is on a boundary between being healthy and being sick. Being small, autonomous, portable, comfortable and easy to use, the integrated system is particularly suitable for long-term monitoring while the individual is undergoing normal life activities (monitoring 'on-the-go)."
3. Noise reduction and signal quality enhancement for body sound signals, including speech, cardiovascular and respiratory sounds, as well as contextual sound signals, including motion-related and environmental sounds;
4. Extraction of multiple sound signals, including speech, physiological sounds and environmental sounds, from a combination of sensors, where each individual sensor provides information resulting from a superposition of many (at least two) physiological signals;
5. Combine body sounds from multiple sources, in order to monitor the health status of an individual, where health status includes physiological, psychological and psycho-physiological components.

Hence, it is an aim of the invention to solve one or more, optionally all, of said problems 1-5.

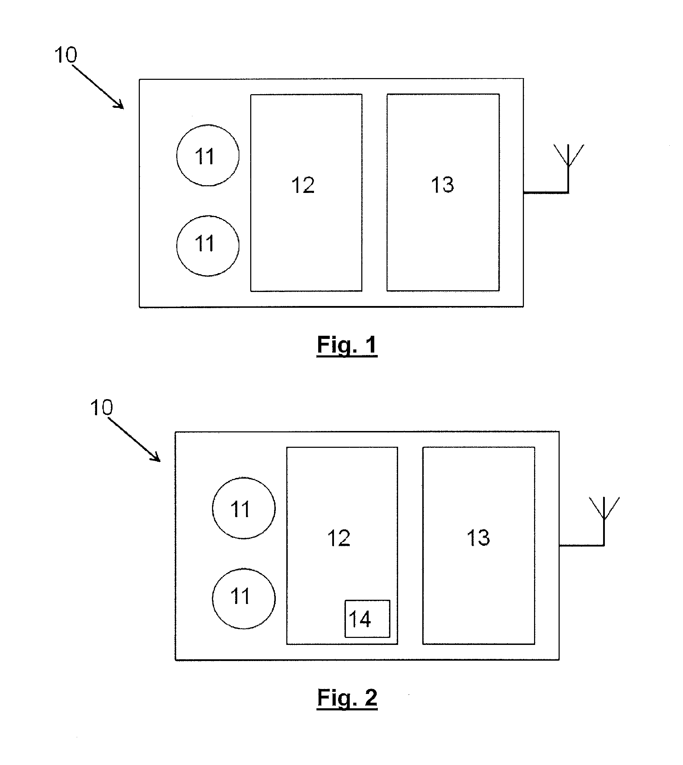

Figure 1 shows a body sound monitoring system according to the invention. With a monitoring system a system is meant that may be capable of checking, observing, examining, recording and processing. The system (10) comprises a number of microphones (11) each arranged for recording a mix of body sounds. In an embodiment, this number of microphones comprises several identical microphones. For example, the system comprises a number of acoustic microphones. In another embodiment, the number of microphones comprises different types of microphones. For example, the system comprises at least one acoustic microphone and at least one contact microphone. The system may further comprise sensing means arranged for sensing physiological signals such as biopotentials, tissue impedance, temperature and any other parameter that may be measured and used to estimate or predict the health condition of the user or help to improve the quality of other signals.

The system further comprises a processing block (12) arranged for receiving the mix of body sounds and for (pre-)processing these signals. The system may also comprise a radio (13) arranged for transmitting data relating to the (pre-)processed mix of body sounds to an external device (such as a processor, cell phone, display unit...). For example, the data is transmitted to a processor for further processing and analyzing the body sounds. Alternatively, the system may comprise a means for storing the body sounds. The processing block (12) may for example be arranged for filtering or transforming the recorded body sounds (transforming to component space). In particular, the processing block may be arranged for performing any of the following actions: filtering, wavelet transformation, beamforming, acoustic holography or any other processing activity required by the application. Further aspects of the processing and analysing may be found in: "Grundlehner, B.; Buxi, D.; , "Methods to Characterize Sensors for Capturing Body Sounds," Body Sensor Networks (BSN), 2011 International Conference on, pp. 59-64, 23-25 May 2011, Dallas TX, doi: 10.1109/BSN.2011.22, print ISBN: 978-1-4577-0469-7", the contents of which is included herein by reference.

In an embodiment, there is provided an integrated body sound monitoring system arranged for recording and processing body sound signals, e.g. representing a respiratory sound. The system may comprise a microphone arranged for recording a body sound signal that represents a mixture of different body sounds. The system may further comprise the processing block that is arranged for processing the body sound signal and comprises electronic components for locally storing, processing and analyzing the body sound signal, preferably prior to transmittal to an external device. The system may further comprise a radio that is arranged for transmitting data related to the body sound signal to the external device. Hence, as a result of the radio, complicated wiring may be prevented in the system.

Capturing body sounds usually involves picking up mechanical vibrations at the skin. A conversion of the mechanical information into an electrical signal may be accomplished by the microphone. Placing a transducer at the body part tends to influence the vibrations at the body part. Mechanical loading of the tissue by the microphone and may result in uncontrolled distortion or damping of the vibrations. Vibrations at the skin due to body sounds are typically very small in amplitude. E.g. for heart sounds at the thorax, in acceleration terms, the order of magnitude of the peak value may be approximately 1 m/s². An amplitude spectrum may decrease progressively with increasing frequency. Hence, reliable recording of body sounds may be problematic.

The microphone may comprise a piezoelectric element that comprises polyvinylidene fluoride, and preferably is substantially made of polyvinylidene fluoride. The piezoelectric material may be formed as a sheet. Such a piezoelectric element may be light and sensitive enough for reliably recording of body sounds. Furthermore, such an element may be flexible. Such flexibility enables placement of the microphone in a close vicinity of, or at a position on a human body. Additionally, such a piezoelectric element may require a relatively low power, as excitation and/or trivial signal conditioning may not be needed. Also, by means of a microphone comprising said piezoelectric element, sounds can be recorded over a relatively broad frequency range.

The microphone may be provided with leads. Deformation of the piezoelectric element, caused by the mixture of body sounds, may lead to a potential difference at the leads. In order to read out said potential difference, the electronic components of the processing block may for example form a non-inverting amplifier, e.g. with a gain of 2. The amplifier's output may be passed through a 2^{nd} order Butterworth low pass filter formed by the electronic components. Said filter may e.g. have a cut-off frequency of approximately 1.3 kHz. The filter's output may be buffered before being fed to a data acquisition card in order to reduce any electromagnetic interference. The processing block may be arranged for digitizing the recorded body sound mixture. A sampling frequency may be set to 10 kHz. The input dynamic range may be set to -5 to +5 V, digitized with 16 bits ADC. It may be DC coupled. However, alternatively, another gain, dynamic range, etc. than described in this example may be used.

Figure 2 illustrates a body sound monitoring system wherein the processing block (12) further comprises an analyzing block (14) arranged for analyzing the mix of body signals or, in other words, the mix of body sounds. The processing block (12) typically comprises electronic components for locally storing, processing and analyzing the body sounds, or, in other words, the body sound signals. The analyzing block (14) may further comprise means for data fusion and means for analyzing health conditions. With data fusion is meant that the information from several parts of the system is recombined, or fused, in order to make a more robust estimation on a higher level. Examples are (from low to high abstraction): a combination of several sensor inputs (potentially from several patches) to reduce the impact of noise, recombination of processed body sounds, or in other words, processed body sounds signals, (e.g. heart and lung sounds) for multi-modal health analysis or combining events from several patches to enhance precision (positive predictivity). Examples of actions to be performed by the means for data fusion are, but not limited to: adaptive filtering with a reference input, pattern recognition, heart sound analysis in conjunction with breathing cycle state (for example for heart rate variability analysis), combined speech and heart rate variability analysis for emotion monitoring. The means for analyzing health conditions analyzes the different retrieved body sounds, or, in other words, retrieved body sound signals, such as: heart sounds, lung sounds, speech, gastrointestical tract... The outcome of the health analysis can be used to provide feedback on the general health status of the individual being monitored. Furthermore, it can be used to monitor for specific health conditions or diseases. From the heart sounds different analyses can be made: heart rate, valve stiffening, valve damage, closure of interatrial septum, detection of murmurs (many pathologies). From the lung sounds it is possible to make an asthma diary and prediction, or do COPD monitoring or learn something of a heart failure. Heart and lungs can be analyzed in combination for more accurate diagnosis. Emotions may be monitored via speech, heart rate and/or breathing rate. Sounds from the gastrointestical tract may be useful as post-operative anaesthesia monitoring.

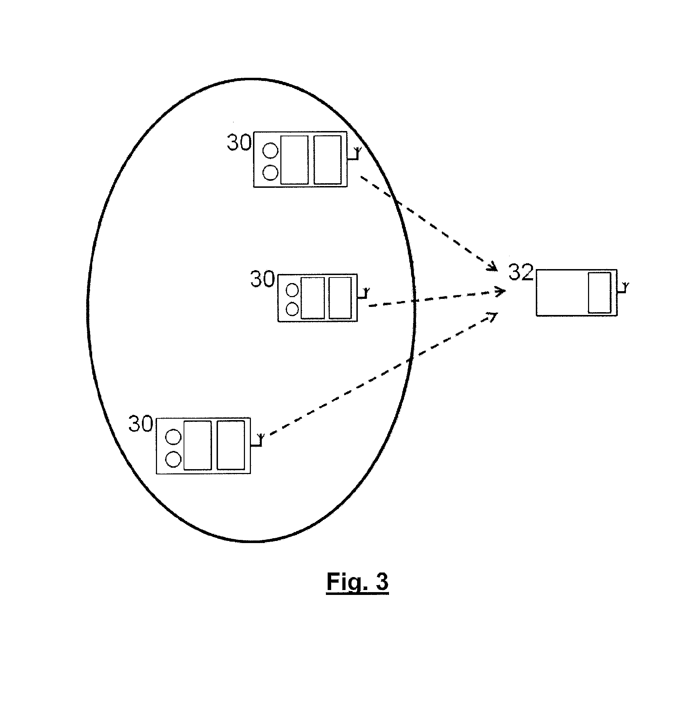

The body sound monitoring system is typically arranged as a patch to be placed in the close vicinity of a person. With a patch an adhesive wearable device is meant that can be applied on the skin in order to sense, record and/or process signals that are generated by the body of the wearer and/or his/her surrounding. A network of these patches may be formed, as in a body area network (BAN) conformation. An example of a network is illustrated in Figure 3. Several body sound monitoring systems or patches (30) are placed at several places of a human body. Each patch (30) senses the mix of body sounds and digitizes it. Each patch is intelligent in the way that it performs some level of processing, analog or digital, on the sensed data. The recorded and (pre-)processed mix of body sounds of each patch is transmitted to an external device (32) such as a central control unit, a processor or a cell phone.

In case environmental, context or motion artifacts are expected to have an important contribution, the BAN can include a third patch dedicated to monitor environment-, context- or motion-related sounds. Preferably, this will be achieved by a microphone capturing body-borne signals, placed on such a location, that the speech signal, respiratory sounds and cardio-vascular sounds are not captured or at least not with high attenuation. Alternatively, the information about motion-related artifacts will be captured by means of other movement monitoring techniques, such as accelerometers for instance.

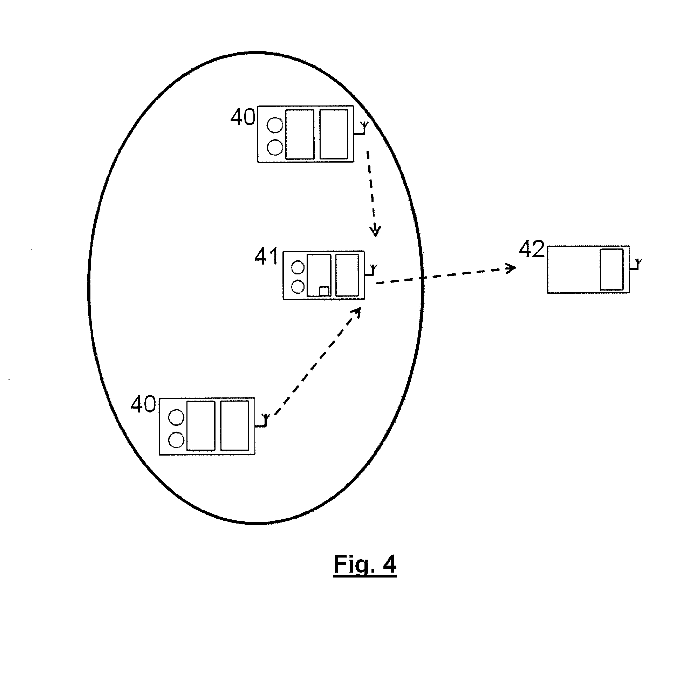

Figure 4 illustrates another example of a network using the body sound monitoring system. Again, a number of patches (40) are placed in the close vicinity of a person. In this example, a more "intelligent" body sound monitoring system (41) is also placed at the human body. This more "intelligent" body sound monitoring system comprises an analyzing block as presented previously and is arranged for receiving data coming from other patches (40). The analyzing block will combine its own recorded and processed mix of body signals with the received signals. Processed and analyzed data may further be transmitted to an external device (42). Sound (pre-)processing and health analysis is thereby performed in the network.

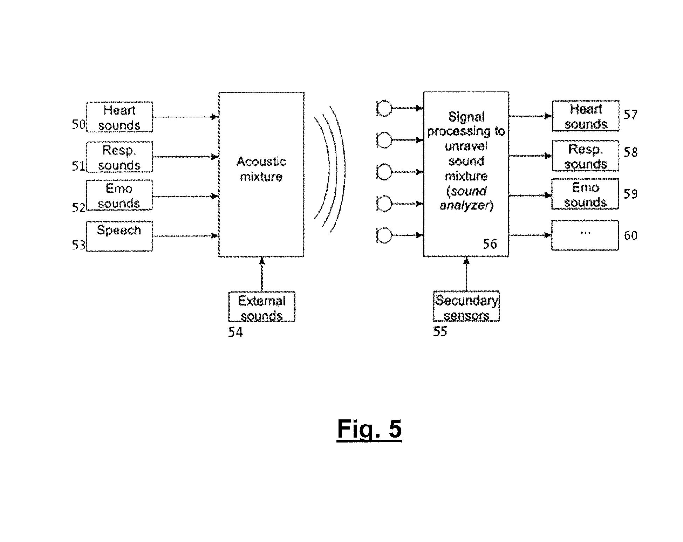

In the previous embodiments a mixture of recorded (or sensed) sounds are used to extract and improve the quality of the separate sound sources. With a holistic approach, an entire map of body sounds can be extracted from many individual sounds. This requires a multitude of recordings, preferably positioned at several places on the body, each recording having a unique and independent mixture of body sounds, or, in other words, of body sound signals. Separate signals can be reconstructed by the use of proper signal processing techniques. Figure 5 shows an illustration of this approach. The system recombines several sensors (50), (51), (52), (53), (54), (55) (can be of different types and can be combined in a single patch or in several patches), which enables the different body sounds (57), (58), (59), (60) to be separated in order to construct a sound 'scene', being a constellation of multiple signals coming from multiple sources. As such, the system may acts as a "body sound analyzer" (56), providing a complete picture of the body sounds; or as a "body and environment sound analyzer", in case environment sounds are included in the picture.

The sound analyzer creates the sound fingerprint, the complete sound picture for the monitored phenomenon. It separates and extracts the independent sound components out of the signals coming from the sensors. As such, it performs independent signal extraction based on multiple sensor data fusion. These independent sound components include:
- Speech signal
- Cardiovascular signal;
- Respiratory signal
- Abdominal sounds
- Motion-related signal
- Background noise signal
- Residual signal

Speech will be contained in both air-borne and body-borne signals - it is therefore possible to separate background noise and speech in the air-borne signal. The background noise can be used for further analysis as contextual information. The speech signal can be used for further analysis in many applications, such as emotion or speech recognition.

The motion noise can be cancelled in the body-borne sound (alternatively, the dedicated movement sensors), since there is one signal that solely contains information regarding motion. That means that the body-borne sound can be processed to give a signal that mainly carries cardiovascular sounds and respiratory sounds. Signal (post) processing methods can be applied to separate those sounds, as described in literature.

At the output of the sound analyzer, the target signals (sounds) have been cleaned from cross-interference and background noise, thus leading to improved performance of their respective applications. As such, the proposed method already offers an improvement to existing monitoring systems based on sound data. An example would be enhanced cardiovascular monitoring, achieved by accounting for respiratory and motion artifacts.

In a further embodiment, the system also includes a "health monitoring component", which combines the output of the "body sound analyzer' and computes the health status of the individual being monitored. The health status may be of physiological, psychological or psycho-physiological nature. The physiological status may include information on the cardiovascular and respiratory systems. The psycho-physiological information includes information on the emotional status of the individual. This 'health monitor' uses signal processing techniques, data-fusion methods and a computer-based model to infer the health status based on, at least, the body sounds, as extracted from the 'body sound analyzer'. In a further extension, other physiological signals and/or contextual information may be used to compute the health status for example based on data-fusion. In a first example, the system can be used to monitor the physiological health of the individual being monitored, integrating, amongst others, aspects related cardiovascular, respiratory and physical activity. In a second example, the system can be used to monitor the psycho-physiological status of the individual being monitored. Emotion monitoring, for example, can be enhanced with information of context, heart beat and respiration, the latter two compensated for motion.

It may be clear from the above description, in particular the description with reference to figures 1-5, that, in an embodiment, a method is presented for monitoring a body sound, by means of an integrated body sound monitoring system 10 according to the invention. The method may comprise providing an integrated body sound monitoring system 10 arranged for recording and processing body sound signals. The method may comprise recording a body sound signal that represents a mixture of different body sounds, by means of a number of microphones 11, e.g. at least one microphone 11 or a plurality of microphones 11, comprised by the system 10. The method may comprise, by means of a processing block 12 comprised by the system 10, processing the body sound signal. The method may comprise locally storing, processing and/or analyzing the body sound signal by means of electronic components of the processing block 12, preferably prior to transmittal to an external device 32, 42. The method may further comprise transmitting, by means of a radio 13 comprised by the system, data related to the body sound signal to the external device 32, 42.

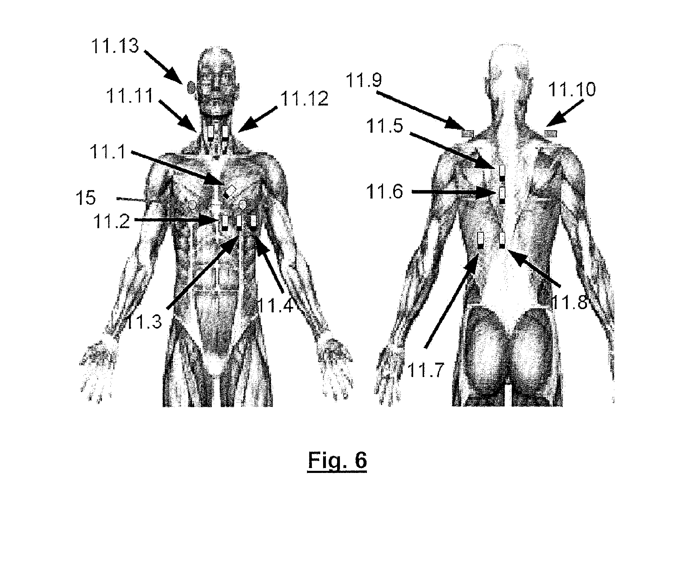

In an embodiment, there is provided a method of placement of a plurality of integrated body sound monitoring systems 10. Figure 6 schematically shows the plurality of systems 10 placed on a human body. The systems may be placed in a close vicinity of, or at, mutually different positions of a human body.

More in general, the systems may, in use, be placed e.g. on a skin of the human body. Each of the systems may comprise at least one microphone 11.i (i=1, ..., 12) arranged for recording a body sound signal that represents a mixture of different body sounds. Each system may further comprise a processing block that is arranged for processing the body sound signal and comprises electronic components that are arranged for locally storing, processing and analyzing the body sound signal prior to transmittal to an external device. Adhesive tape may be used to attach the microphones 11.i to the skin. Each system may further comprise a radio that is arranged for transmitting data related to the body sound signal to the external device.

Said method may comprise at least one, at least two, or at least three, preferably four, steps of the following group of four steps.

A first step may comprise placing the at least one microphone, e.g. four microphones 11.1-11.4, of at least one of the systems at a position that is suitable for substantially recording a heart sound. Preferably, said location is in a vicinity of a heart of the human body, e.g. on a chest of the human body. The microphones 11.1-11.4 may be comprised by one, two, three, or four integrated body monitoring systems 10. Microphones 11.1-11.4 may e.g. be included in a single patch.

A second step may comprise placing the at least one microphone, e.g. six microphones 11.5-11.10, of at least a second one of the systems at a position that is suitable for substantially recording a lung sound. Preferably, said position is in a vicinity of a lung of the human body, e.g. on a back of the human body. The microphones 11.5-11.10 may be comprised by one, two, three, four, five, or six integrated body monitoring systems 10. Microphones 11.5-11.8 may e.g. be included in a single patch. Further, microphone 11.9 may be included in a single patch and microphone 11.10 may be included in a single patch.

A third step may comprise placing at least one microphone, e.g. three microphones 11.11-11.13, of at least a third one of the systems at a position that is suitable for substantially recording a speech sound, e.g. on a neck, in particular on a throat, or in an ear of the human body. Preferably, said position is in a vicinity of a throat of the human body. The microphones 11.11-11.13 may be comprised by one, two, or three integrated body monitoring systems 10. One of the microphones placed for substantially measuring a speech sound may be placed near or in an ear of the human body. Microphones 11.11 and 11.12 may be included in a single patch.

A fourth step may comprise placing at least one microphone of at least a fourth one of the systems at a position that is suitable for substantially recording a gastrointestical tract sound. Preferably, said position is in a vicinity of an abdomen of the human body, e.g. on the abdomen of the human body.

Preferably, the method comprises at least the first step, the second step, and the third step.

The placement according to the first, second, third and/or fourth step may enable recording of at least three body sound signals that each represent a mixture of body sounds. In the example of placement illustrated in figure 6, placement according to the first step may be dedicated to recording a heart sound, placement according to the second step may be dedicated to recording a lung sound, placement according to the third step may be dedicated to recording a speech sound, and placement according to the fourth step may be dedicated to recording an gastrointestical tract sound. Thus, as a result of the placement, each mixture may be dedicated to a specific body sound, i.e. may be dedicated to the heart sound, the lung sound, the speech sound, the gastrointestical sound, or another body sound, so that that specific body sound can be substantially, e.g. predominantly, recorded. With the term 'substantially' it may be meant that a body sound signal, or data related thereto, that represents a specific body sound can be separated from, or identified in, the recorded body sound signal. Hence, the placement enables a combination of recording of different body sounds. Experiments carried out by the inventors showed that a quality of recorded sound may be enhanced by placement according to said method.

In this embodiment, the recording of speech sound may be enabled by means of the microphones 11.11-11.13. It was recognized that recording of speech sound may improve and/or enable an analysis of an emotion of a person. In particular, the combination of the recording of the speech sound with the recording of the heart sound and the lung sound may enable an analysis of said person with a relatively high quality.

The method may further comprise recording the heart sound by means of the microphones 11.1-11.4 placed for substantially recording the heart sound, may comprise recording the lung sound by means the microphones 11.5-11.10 placed for substantially recording the lung sound, may comprise recording the speech sound by means of the microphones 11.11-11.13 placed for substantially recording the speech sound, and/or may comprise recording the gastrointectical sound by means of the at least one of the microphones placed for substantially recording the gastrointestical tract sound. The method may further comprise, by means of processing blocks of the systems, storing and processing the recorded body sound signals, said processing comprising combining at least two, preferably at least three, recorded body sound signals for reducing noise and/or enhancing quality of the body sound signals.

Microphones 11.1-11.8, 11.11, and 11.12 may e.g. have a piezoelectric element that comprises polyvinylidene fluoride. Microphones 11.1-11.8 and 11.11, and 11.12 may be regarded as examples of contact microphones. Microphones 11.9, 11.10, and 11.13 may be other type of microphones, e.g. conventional microphones. Microphones 11.9, 11.10, and 11.13 may be regarded as examples of acoustic microphones.

In a variation, the method may comprise measuring an ECG (Electro Cardiograph) and/or thoracic expansion, optionally in parallel with recording body sound signals by means of the microphones. ECG and/or thoracic expansion may be carried out e.g. by means of a belt 15. The belt 15 may comprise electrodes for ECG measurements. The measurements of the ECG and/or the measurements of the thoracic expansion may be carried out synchronously with recording the body sound signals. Thus, measurements of the ECG and/or thoracic expansion may be synchronized with recording of a body sound signal, e.g. a heart sound signal. By means of such synchronization, the measurement of ECG and/or thoracic expansion can be better compared with the recording of a body sound signal. Alternatively or additionally, a start time of recordings of the microphones of the systems may be mutually synchronized.

More in general, a method according to the invention may, preferably, comprise synchronising the recording of body sound signals. Thus, recorded body sound signals may be mutually synchronised. The external device may e.g. be arranged for carrying out the synchronisation. Thereto the external device may e.g. be arranged to send a signal to each microphone of the number of microphones to start recording. Sending such a signal may alternatively be carried out by means of a base station that is separate from the external device. Alternatively, sending the signal may be carried out by means of a processing block of a system.

In summary, a system is provided having the following advantages over the prior art:
1. By combining multiple (at least two) acoustic sensors, the proposed system and method combine redundant information to extract an enhanced body sound signal, cleaned from cross-interference and background noise. Therefore, the proposed method enhances existing noise reduction techniques based on signal post-processing.
2. By merging multiple (at least two) sensors, the proposed system and method separates independent body sounds out of sensor signals, where independent body sounds correspond to different physiological processes (cardiovascular, respiratory, speech, motion, others) and where sensor signals results from a superposition of these independent body sounds.
3. By extracting a plurality of body sound signals, and combining them using data fusion techniques and a health monitoring model, the proposed system and method implements a new sound-based health monitoring paradigm. The proposed sound-based health monitor may be a physiological health monitor providing information on cardiovascular or respiratory conditions based on physiological sound signals cleaned from motion and speech artifacts. In a further embodiment, the proposed sound-based health monitor may also include a psycho-physiological component, which relies on fusion of the cardiovascular, respiration, speech and contextual signals, previously filtered for cross-interference and motion artifacts.
4. Diseases are associated to specific patterns or irregularities in the body sounds. By detecting specific markers, the proposed system allows to monitor the on-set or evolution of diseases over time.

Hence, the system may have one or more, preferably all, of said advantages 1-4.

The previous examples and embodiments are not limited to recording and processing body sounds from human being but can also be used for recording and processing body sounds from other living species or even for recoding and processing sounds coming from machines. Specific sound fingerprints can be due to wearing of material. An obvious example are gear boxes, where analysis of harmonics can be used to track wear of the separate parts (useful in machines, cars, windmills, ...). Another example are bridges, where vibrations with a certain character at a certain intensity might be related to wear, or similarly the behavior of wings of airplanes. Another example is road-joints (e.g. where roads and viaducts/bridges connect), which are today monitored manually on a regular basis.

## Claims

1. Integrated body sound monitoring system arranged for recording and processing body sound signals, wherein the system comprises:
- a number of microphones arranged for recording a body sound signal that represents a mixture of different body sounds;
- a processing block that is arranged for processing the body sound signal and comprises electronic components for locally storing, processing and analyzing the body sound signal prior to transmittal to an external device; and
- a radio that is arranged for transmitting data related to the body sound signal to the external device.

2. System according to claim 1, wherein the mixture of body sounds comprises at least three sounds of the group comprising a heart sound, a lung sound, a speech sound and a gastrointestical tract sound.

3. System according to claim 1 or 2, wherein the processing block comprises an analyzing block for analyzing the body sound signal prior to transmittal to the external device.

4. System according to one of claims 1-3, wherein the analyzing block comprises means for data fusion arranged for combining a plurality of recorded body sound signals from the microphones, and preferably further comprises means for analyzing health conditions arranged for analyzing the recorded body sound signals.

5. System according to one of claims 1-4, wherein at least one of the number of microphones comprises a piezoelectric element that comprises polyvinylidene fluoride.

6. System according to one of claims 1-5, wherein the number of microphones comprises at least one acoustic microphone and at least one contact microphone.

7. System according to one of claims 1-6, further comprising sensing means arranged for sensing at least one physiological signal such as a biopotential, a tissue impedance, and/or a temperature.

8. System according to one of claims 1-7, arranged for providing the user with physiological and psychological health information extracted from a multiple of recorded body sound signals.

9. System according to one of claims 1-8, arranged as a patch arranged for placement in a close vicinity of a person.

10. System according to one of claims 1-9, being a distributed system comprising a plurality of integrated body sound monitoring units according to one of claims 1-9, said units being arranged for placement in a close vicinity of a person, preferably for placement at several positions of a human body, and for forming a network, wherein each unit, in use, records at least one body sound signal that represents a mixture of body sounds and processes, e.g. digitizes, the at least one body sound signal, wherein, in use, the recorded and processed body sound signals of each body sound system are transmitted to the external device.

11. System according to claim 10, wherein at least one of the units is arranged to monitor environment-, context- or motion-related sounds, by having, in use, a microphone placed on such a location that a speech sound, a lung sound, and a cardio-vascular sound are not recorded or at least not recorded with a high attenuation.

12. System according to claim 10 or 11, wherein a unit of the plurality of units is arranged for receiving data coming from other units of the plurality of units, and comprises an analyzing block that is comprised by the processing block, the analyzing block being arranged for combining the recorded and processed body sound signals by said unit of the plurality of units with the received signals from other units of the plurality of units.

13. Method for monitoring body sounds, by means of an integrated body sound monitoring system according to one of claims 1-12.

14. Method of placement of a plurality of integrated body sound monitoring systems in a close vicinity of, or at, mutually different positions of a human body, wherein each of the systems comprises at least one microphone arranged for recording a body sound signal that represents a mixture of different body sounds, and further comprises a processing block that is arranged for processing the body sound signal and comprises electronic components that are arranged for locally storing, processing and analyzing the body sound signal prior to transmittal to an external device, and further comprises a radio that is arranged for transmitting data related to the body sound signal to the external device, wherein the method comprises at least three, preferably four, steps of the following group of four steps:
- placing the at least one microphone of at least one of the systems at a position, preferably in a vicinity of a heart of the human body, that is suitable for substantially recording a heart sound;
- placing the at least one microphone of at least one of the systems at a position, preferably in a vicinity of a lung of the human body, that is suitable for substantially recording a lung sound;
- placing the at least one microphone of at least one of the systems at a position, preferably in a vicinity of a throat of the human body, that is suitable for substantially recording a speech sound; and
- placing the at least one microphone of at least one of the systems at a position, preferably in a vicinity of an abdomen of the human body, that is suitable for substantially recording a gastrointestical tract sound.

15. Method according to claim 14, further comprising recording the heart sound by means of the at least one microphone of the at least one the systems placed at the position that is suitable for substantially recording the heart sound, comprising recording the lung sound by means of the at least one microphone of the at least one the systems placed at the position that is suitable for substantially recording the lung sound, comprising recording the speech sound by means of the at least one of the microphones of the at least one the systems placed at the position that is suitable for substantially recording the speech sound, and/or comprising recording the gastrointectical sound by means of the at least one of the microphones of the at least one of the systems placed at the position that is suitable for substantially recording the gastrointestical tract sound.
